# EUROPÄISCHE PATENTSCHRIFT

(11) **EP 0 628 606 B1**
(45) Veröffentlichungstag und Bekanntmachung des Hinweises auf die Patenterteilung: **23.08.2000**
(21) Anmeldenummer: 94106830.6
(22) Anmeldetag: 02.05.1994
(51) Int. Cl.: C09B 62/002, C09B 62/04, C09B 62/503, C09B 67/22

(54) **Triphendioxazinverbindungen, Verfahren zu ihrer Herstellung und ihre Verwendung als Farbstoffe**
Triphendioxazine compounds, process for their preparation and their use as dyes
Composés triphènedioxazine, procédé pour leur préparation et leur utilisation comme colorants

(30) Priorität: 18.05.1993 DE 4316539; 02.02.1994 DE 4403065
(43) Veröffentlichungstag der Anmeldung: 14.12.1994
(73) Patentinhaber: DyStar Textilfarben GmbH & Co. Deutschland KG, 60318 Frankfurt am Main (DE)
(72) Erfinder: Russ, Werner Hubert, Dr., D-65439 Flörsheim/Main (DE); Tappe, Horst, Dr., D-63128 Dietzenbach (DE); Schumacher, Christian, Dr., D-65929 Frankfurt am Main (DE)

(56) Entgegenhaltungen:
- EP-A- 0 499 588
- EP-A- 0 568 860

## Beschreibung

Die Erfindung liegt auf dem technischen Gebiet der faserreaktiven Farbstoffe.

Asymmetrisch aufgebaute faserreaktive Triphendioxazinfarbstoffe, d.h. Triphendioxazinfarbstoffe, deren einer endständiger Benzolkern eine Aminogruppe und deren anderer endständiger Benzolkern eine 2-Halogen-4-[N-phenyl-N-β-(β'-sulfatoethylsulfonyl)-ethyl]-amino-1,3,5-triazin-6-yl-amino-Gruppe oder Dichlor-triazinylamino-disulfophenylamino-chlortriazinylamino-Gruppe enthält, sind in der europäischen Patentanmeldungs-Veröffentlichung Nr. 0 568 860 bzw. der britischen Patentschrift Nr. 1 349 513 beschrieben.

Mit der vorliegenden Erfindung wurden neue Triphendioxazin-Verbindungen entsprechend der allgemeinen Formel (1) 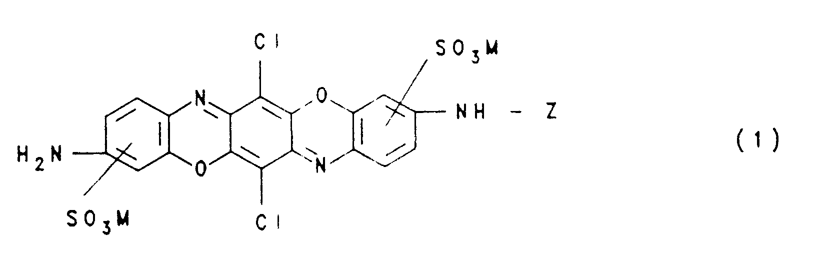 gefunden, in welcher bedeuten:
- M: ist Wasserstoff oder ein Alkalimetall, wie Natrium, Kalium oder Lithium;
- Z: ist ein Rest der allgemeinen Formel (2) 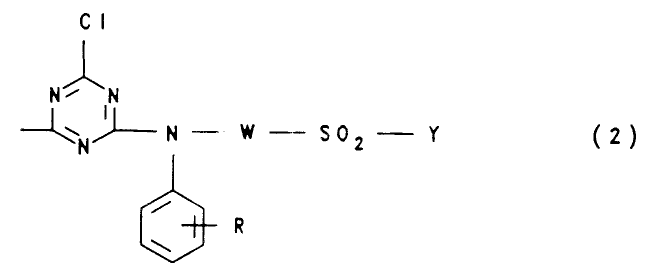
in welcher
- R: Wasserstoff, Sulfo, Alkyl von 1 bis 4 C-Atomen, wie Ethyl und insbesondere Methyl, Alkoxy von 1 bis 4 C-Atomen, wie Ethoxy und insbesondere Methoxy, Nitro oder Cyano ist,
- W: Alkylen von 3 bis 6 C-Atomen bedeutet, bevorzugt Isopropylen und insbesondere n-Propylen ist, und
- Y: Vinyl ist oder Ethyl ist, das in β-Stellung durch einen bei Einwirkung von Alkali unter Bildung der Vinylgruppe eliminierbaren Substituenten substituiert ist;
die beiden Sulfogruppen -SO₃M stehen bevorzugt in ortho-Stellung zum Sauerstoffatom des heterocyclischen Ringes an den Benzolkern gebunden.

β-ständige Substituenten in der Ethylgruppe Y sind beispielsweise Sulfato, Thiosulfato, Phosphato, Alkanoyloxy von 2 bis 5 C-Atomen, wie Acetyloxy, Benzoyloxy, Sulfobenzoyloxy, p-Toluolsulfonyloxy oder Halogen, wie Brom oder Chlor. Bevorzugt ist Y Vinyl, β-Chlorethyl und insbesondere β-Sulfatoethyl. Weiterhin bevorzugt ist R gleich Wasserstoff oder Sulfo und insbesondere bevorzugt Wasserstoff.
Reste W sind beispielsweise außer dem besonders bevorzugten 1,3-Propylen das 2-Methyl-1,2-ethylen, 1-Methyl-1,2-ethylen, 2-Methyl-1,3-propylen, 1,4-Butylen, 1,5-Pentylen und 1,6-Hexylen.

Von den erfindungsgemäßen Triphendioxazin-Verbindungen der allgemeinen Formel (1) können insbesondere diejenigen hervorgehoben werden, die der allgemeinen Formel (1A) 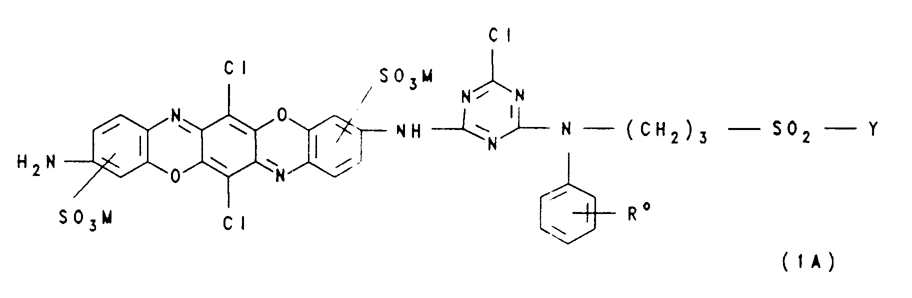 entsprechen, in welcher M und Y die obengenannten, insbesondere bevorzugten Bedeutungen haben und R^{o} gleich Wasserstoff oder Sulfo ist.

In den allgemeinen Formeln (1) und (2) sowie in den nachfolgenden allgemeinen Formeln können die einzelnen Formelglieder, sowohl verschiedener als auch gleicher Bezeichnung innerhalb einer allgemeinen Formel, im Rahmen ihrer Bedeutung zueinander gleiche oder voneinander verschiedene Bedeutungen haben.

Die Gruppen "Sulfo", "Carboxy", "Phosphato", "Thiosulfato" und "Sulfato" schließen sowohl deren Säureform als auch deren Salzform ein. Demgemäß bedeuten Sulfogruppen Gruppen entsprechend der allgemeinen Formel -SO₃M , Carboxygruppen Gruppen entsprechend der allgemeinen Formel -COOM , Phosphatogruppen Gruppen entsprechend der allgemeinen Formel -OPO₃M₂ , Thiosulfatogruppen Gruppen entsprechend der allgemeinen Formel -S-SO₃M und Sulfatogruppen Gruppen entsprechend der allgemeinen Formel -OSO₃M , in welchen M die obengenannte Bedeutung hat.

Die vorliegende Erfindung betrifft weiterhin ein Verfahren zur Herstellung der erfindungsgemäßen Triphendioxazin-Verbindungen der allgemeinen Formel (1), das dadurch gekennzeichnet ist, daß man eine aminogruppenhaltige Dichlortriazinylamino-triphendioxazin-Verbindung der allgemeinen Formel (3) 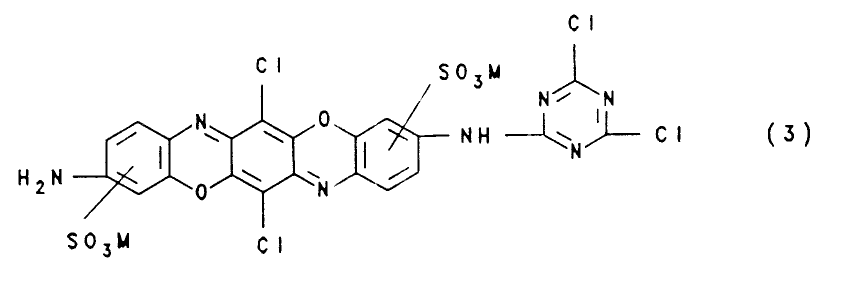 mit M der obengenannten Bedeutung in wäßrigem oder wäßrig-organischem Medium bei einem pH-Wert zwischen 3 und 9, bevorzugt zwischen 4 und 5, und bei einer Temperatur zwischen 25 und 100°C, bevorzugt zwischen 40 und 80°C, mit einer Aminoverbindung der allgemeinen Formel (4) 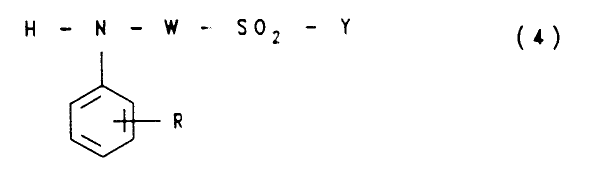 in welcher R, W und Y die obengenannten Bedeutungen haben, umsetzt.

Führt man die Umsetzung in einem wäßrig-organischen Medium durch, so ist das organische Medium ein gegenüber den Reaktanten inertes, dipolar aprotisches Lösemittel, das in Wasser löslich oder mit Wasser mischbar ist, wie beispielsweise Aceton, Dimethylformamid, Dimethylacetamid, Sulfolan oder N-Methyl-pyrrolidon. Vorteilhaft wird bei der Kondensation freiwerdende Salzsäure laufend durch Zugabe wäßriger Alkali- oder Erdalkalihydroxide, -carbonate oder -bicarbonate neutralisiert.

Die Herstellung der Ausgangsverbindung der allgemeinen Formel (3) ist an und für sich bekannt (vgl. europäische Patentanmeldungs-Veröffentlichung Nr. 0 448 815, Beispiel 1, europäische Patentanmeldungs-Veröffentlichung Nr. 0 142 777, Beispiel 44, und deutsche Auslegeschrift Nr. 2 124 080, Beispiel 1). So kann die Umsetzung zwischen der Diamino-triphendioxazin-Verbindung und dem Cyanurchlorid bei einem pH-Wert zwischen etwa 4 und 7 und einer Temperatur zwischen etwa 0 und 25°C erfolgen.

Die Ausgangsverbindungen der allgemeinen Formel (4) sind bisher noch nicht beschrieben. Sie lassen sich beispielsweise in der Weise herstellen, daß man N-Allyl-N-acetyl-anilin (s. J. Org. Chem. 14, 1099 (1949)) analog der in der deutschen Offenlegungsschrift Nr. 41 06 106 beschriebenen Verfahrensweise mit 2-Mercapto-ethanol in Gegenwart eines Radikalinitiators umsetzt, die erhaltene N-[γ-(β'-Hydroxyethylthio)-propyl]-N-acetyl-anilin-Verbindung zur Sulfonylverbindung oxidiert, beispielsweise mittels Wasserstoffperoxid in Gegenwart einer katalytischen Menge einer Übergangsmetallverbindung, wie beispielsweise Wolframoxid. Aus der so erhaltenen Sulfonylverbindung wird die Acetylgruppe im alkalischen oder sauren Bereich, vorzugsweise in salzsaurer wäßriger Lösung, wie beispielsweise in 5 bis 30 %iger wäßriger Salzsäure, bei einer Temperatur zwischen 80 und 100°C hydrolytisch abgespalten. Das so erhaltene N-Phenyl-N-[γ-(β'-hydroxyethylsulfonyl)-propyl]-amin kann aus der neutral gestellten wäßrigen Syntheselösung von der wäßrigen Phase abgetrennt werden. Dessen β-Hydroxyethylsulfonyl-Gruppe läßt sich nach üblichen Methoden verestern, so beispielsweise mittels konzentrierter Schwefelsäure bei einer Temperatur zwischen 10 und 30°C in die β-Sulfatoethylsulfonyl-Verbindung oder mit Thionylchlorid oder gasförmigem Chlorwasserstoff in die β-Chlorethylsulfonyl-Verbindung überführen.

Ausgangsverbindungen der allgemeinen Formel (4) sind beispielsweise N-Phenyl-N-[γ-(β'-chlorethylsulfonyl)-propyl]-amin, N-(4-Chlorphenyl)-N-[γ-(β'-chlorethylsulfonyl)-propyl]-amin, N-(2-Methylphenyl)-N-[γ-(β'-chlorethylsulfonyl)-propyl]-amin, N-(4-Methoxyphenyl)-N-[γ-(β'-chlorethylsulfonyl)-propyl]-amin, N-(3-Sulfophenyl)-N-[γ-(β'-chlorethylsulfonyl)-propyl]-amin, N-(4-Sulfophenyl)-N-[γ-(β'-chlorethylsulfonyl)-propyl]-amin, N-Phenyl-N-(γ-vinylsulfonyl-propyl)-amin, N-(4-Chlorphenyl)-N-(γ-vinylsulfonyl-propyl)-amin, N-(2-Methylphenyl)-N-(γ-vinylsulfonyl-propyl)-amin, N-(4-Methoxyphenyl)-N-(γ-vinylsulfonyl-propyl)-amin, N-(3-Sulfophenyl)-N-(γ-vinylsulfonyl-propyl)-amin, N-(4-Sulfophenyl)-N-(γ-vinylsulfonyl-propyl)-amin, N-Phenyl-N-[γ-(β'-sulfatoethylsulfonyl)-propyl]-amin, N-(4-Chlorphenyl)-N-[γ-(β'-sulfatoethylsulfonyl)-propyl]-amin, N-(2-Methylphenyl)-N-[γ-(β'-sulfatoethylsulfonyl)-propyl]-amin, N-(4-Methoxyphenyl)-N-[γ-(β'-sulfatoethylsulfonyl)-propyl]-amin, N-(3-Sulfophenyl)-N-[γ-(β'-sulfatoethylsulfonyl)-propyl]-amin, N-(4-Sulfophenyl)-N-[γ-(β'-sulfatoethylsulfonyl)-propyl]-amin, N-Phenyl-N-[β-methyl-γ-(β'-chlorethylsulfonyl)-propyl]-amin, N-Phenyl-N-[β-ethyl-γ-(β'-chlorethylsulfonyl)-propyl]-amin, N-Phenyl-N-[δ-(β'-chlorethylsulfonyl)-butyl]-amin, N-Phenyl-N-[ε-(β'-chlorethylsulfonyl)-pentyl]-amin und N-Phenyl-N-[β-(β'-chlorethylsulfonyl)-hexyl]-amin, bevorzugt hiervon insbesondere N-Phenyl-[γ-(β'-chlorethylsulfonyl)-propyl]-amin und N-Phenyl-[γ-(β'-sulfatoethylsulfonyl)-propyl]-amin.

Die vorliegende Erfindung betrifft weiterhin Mischungen einer oder mehrerer Triphendioxazinverbindungen der allgemeinen Formel (1) mit einer oder mehreren anderen blaufärbenden Verbindungen (Farbstoffen) an und für sich bekannter Konstitution aus der Disazo-, Kupferformazan-, Triphendioxazin- und Anthrachinon-Reihe. Solche Farbstoffe sind beispielsweise Triphendioxazin-Farbstoffe entsprechend der allgemeinen Formel (I) 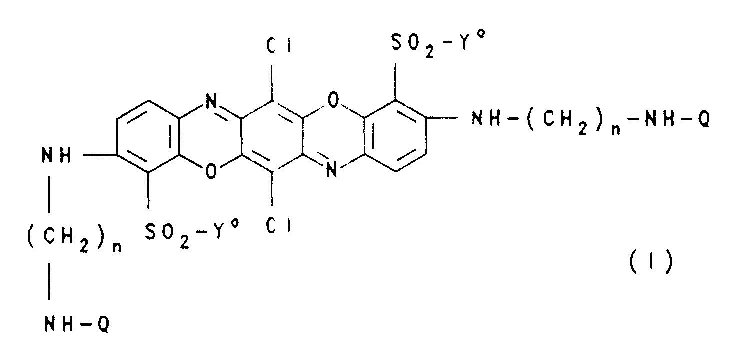 die beispielsweise in den europäischen Patentanmeldungs-Veröffentlichungen Nrs. 0 101 665, 0 153 599 und 0 258 493 beschrieben sind, Anthrachinon-Farbstoffe entsprechend der allgmeinen Formel (II) 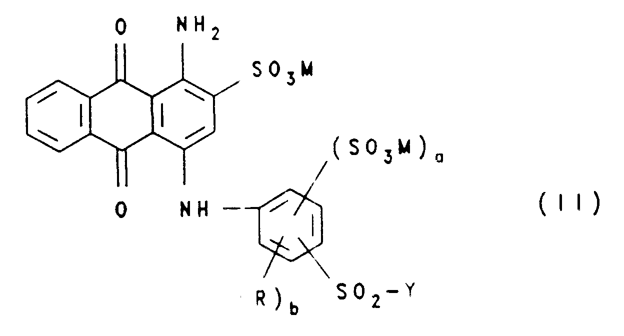 die aus der deutschen Patentschrift Nr. 965 902 bekannt sind, Kupferformazan-Farbstoffe entsprechend der allgemeinen Formel (III) 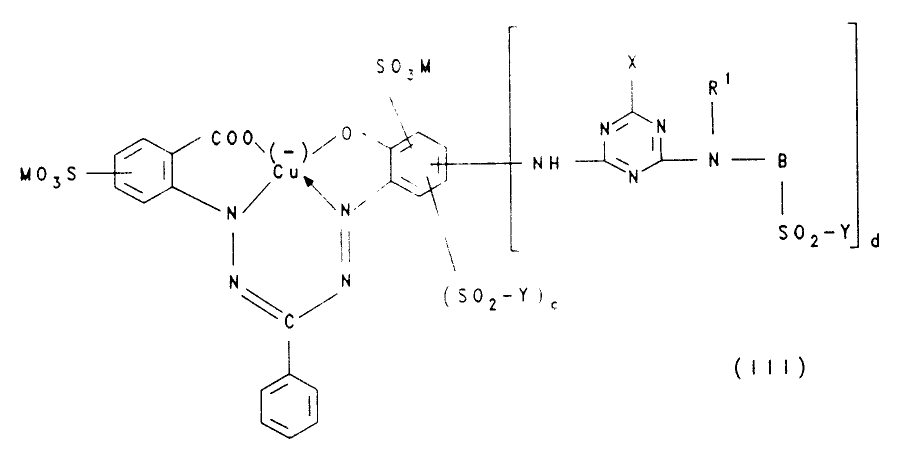 die beispielsweise aus den europäischen Patentanmeldungs-Veröffentlichungen Nrs. 0 028 788 und 0 568 860 bekannt sind, und
Disazofarbstoffe entsprechend der allgemeinen Formel (IV) 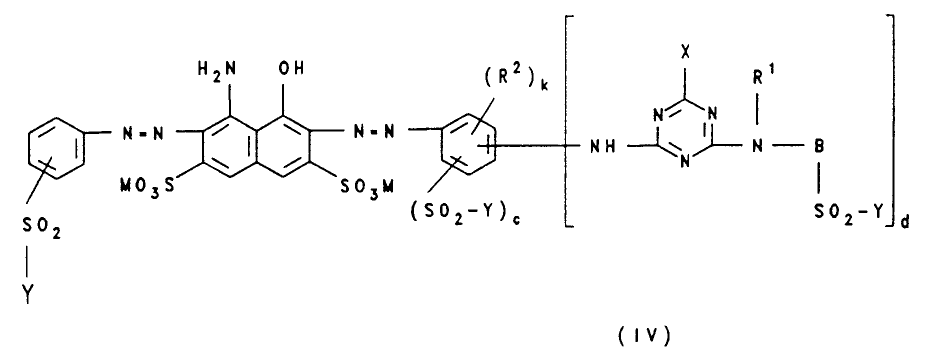 die beispielsweise aus der europäischen Patentanmeldungs-Veröffentlichung Nr. 0 568 860 und aus der deutschen Patentschrift Nr. 965 902 bekannt sind.

In den Formeln (I) bis (IV) bedeuten:
- M und Y: haben eine der obengenannten Bedeutungen;
- n: steht für die Zahl 2 bis 6;
- Y^{o}: ist Hydroxy, Vinyl, β-Halogenoethyl, wie Chlorethyl, oder β-Sulfatoethyl, bevorzugt Hydroxy oder β-Sulfatoethyl;
- Q: ist Hydroxy, Sulfato, Arylamino, Alkylamino mit einem Alkylrest von 1 bis 4 C-Atomen, Dialkylamino mit Alkylresten von jeweils 1 bis 4 C-Atomen, Alkylcarbonylamino von 3 bis 7 C-Atomen, wie β-Carboxypropionylamino, 4-Chlor-6-arylamino-1,3,5-triazin-2-yl-amino, 4-Fluor-6-arylamino-1,3,5-triazin-2-yl-amino oder 4-Cyanoamino-6-arylamino-1,3,5-triazin-2-yl-amino, wobei in diesen Resten der Arylrest für einen Benzol- oder Naphthalinrest steht, der durch 1, 2 oder 3 Substituenten aus der Gruppe Sulfo, Hydroxy, Alkyl von 1 bis 4 C-Atomen, wie Ethyl und Methyl, Alkoxy von 1 bis 4 C-Atomen, wie Ethoxy und Methoxy, β-Sulfatoethylsulfonyl, β-Halogenoethylsulfonyl, wie β-Chlorethylsulfonyl, oder Vinylsulfonyl substituiert sein kann,
wobei im Falle, daß Y für Hydroxy steht, Q einen der genannten Triazinylaminoreste bedeutet, und daß im Falle, daß Y für Vinyl, β-Halogenoethyl oder β-Sulfatoethyl steht, der Rest Q einen der obengenannten Reste mit mindestens einer Sulfogruppe ist;
- R: ist Alkyl von 1 bis 4 C-Atomen, wie insbesondere Methyl;
- a: ist die Zahl Null, 1 oder 2 (wobei im Falle von a gleich Null diese Gruppe Wasserstoff bedeutet);
- b: ist die Zahl Null, 1 oder 2 (wobei im Falle von a gleich Null diese Gruppe Wasserstoff bedeutet);
- c: ist die Zahl Null oder 1 (wobei im Falle von c gleich Null diese Gruppe Wasserstoff bedeutet) und
- d: ist die Zahl Null oder 1 (wobei im Falle von c gleich Null diese Gruppe Wasserstoff bedeutet),
wobei die Summe von (c + d) gleich 1 ist;
- X: ist Chlor oder Fluor;
- k: ist die Zahl 1 oder 2;
- R¹: ist Wasserstoff, Phenyl, das substituiert sein kann, wie beispielsweise durch Sulfo, Alkyl von 1 bis 4 C-Atomen, Alkoxy von 1 bis 4 C-Atomen oder Carboxy, oder ist Alkyl von 1 bis 4 C-Atomen, wie Ethyl und insbesondere Methyl;
- R²: ist Wasserstoff, Sulfo, Alkoxy von 1 bis 4 C-Atomen, wie Ethoxy und insbesondere Methoxy, oder Alkyl von 1 bis 4 C-Atomen, wie Ethyl, insbesondere Methyl, wobei im Falle von k gleich 2 die beiden Substituenten R unterschiedliche Bedeutung besitzen können;
- B: ist ein aliphatisches oder aromatisches Brückenglied, wie beispielsweise Alkylen von 1 bis 6 C-Atomen und Phenylen, das substituiert sein kann, wie beispielsweise durch 1 oder 2 Substituenten aus der Gruppe Sulfo, Methyl, Ethyl, Methoxy, Ethoxy, Carboxy und Nitro, oder ist eine Kombination aus einem aliphatischen und aromatischen Brückenglied.

In der Formel (I) ist im Falle, daß Y β-Sulfatoethyl ist, der Rest Q bevorzugt Sulfato oder β-Carboxypropionylamino und im Falle, daß Y Hydroxy ist, bevorzugt 4-Fluor-6-(2',5'-disulfophenyl)-amino-1,3,5-triazin-2-yl-amino.

In diesen erfindungsgemäßen Mischungen sind der oder die Verbindungen der allgemeinen Formel (1) in der Regel zu 98 bis 30 Gew.-%, bevorzugt zu 96 bis 55 Gew.-%, und der oder die Verbindungen entsprechend den allgemeinen Formeln (I), (II), (III) und/oder (IV) zu 2 bis 70 Gew.-%, bevorzugt zu 4 bis 45 Gew.-%, enthalten.

Die erfindungsgemäße Mischung der Verbindungen der allgemeinen Formel (1) mit einem oder mehreren Verbindungen der allgemeinen Formeln (I) bis (IV) läßt sich üblicherweise durch mechanisches Vermischen der einzelnen Komponenten herstellen. Die Verbindungen der Formeln (I) bis (IV) können jedoch auch in den entsprechenden Mengen der wäßrigen Syntheselösung der erfindungsgemäßen Triphendioxazinverbindungen der allgemeinen Formel (1) zugesetzt werden; in Form dieser wäßrigen Lösung können sie somit, gegebenenfalls nach Aufkonzentrieren oder Verdünnen mit Wasser und gegebenenfalls nach Zusatz einer Puffersubstanz, der färberischen Verwendung zugeführt werden. Der Zusatz von blauen Farbstoffen der allgemeinen Formeln (I) bis (IV) hat den Vorteil, daß man die mit den erfindungsgemäßen Triphendioxazin-Farbstoffen der allgemeinen Formel (1) erhältlichen rotstichig blauen Färbungen leicht in gewünschter Weise nuancieren kann.

Die Abscheidung der erfindungsgemäß hergestellten Verbindungen der allgemeinen Formel (1) aus den Syntheseansätzen erfolgt nach allgemein bekannten Methoden entweder durch Ausfällen aus dem Reaktionsmedium mittels Elektrolyten, wie beispielsweise Natriumchlorid oder Kaliumchlorid, oder durch Eindampfen der Reaktionslösung, beispielsweise durch Sprühtrocknung, wobei dieser Reaktionslösung eine Puffersubstanz zugeführt werden kann.

Die Verbindungen der allgemeinen Formel (1) und die oben beschriebenen erfindungsgemäßen Mischungen eines oder mehrerer der Triphendioxazin-Farbstoffe der allgemeinen Formel (1) mit einem oder mehreren der bekannten Farbstoffe der allgemeinen Formeln (I) bis (IV) - beides im nachfolgenden allgemein als Farbstoffe (1) bezeichnet - eignen sich zum Färben und Bedrucken der verschiedensten Materialien, wie Seide, Leder, Wolle, Polyamidfasern und Polyurethanen, und insbesondere cellulosehaltiger Fasermaterialien aller Art. Solche Fasermaterialien sind beispielsweise die natürlichen Cellulosefasern, wie Baumwolle, Leinen und Hanf, sowie Zellstoff und regenerierte Cellulose. Die Farbstoffe (1) sind auch zum Färben oder Bedrucken von hydroxygruppenhaltigen Fasern geeignet, die in Mischgeweben enthalten sind, zum Beispiel von Gemischen aus Baumwolle mit Polyesterfasern oder Polyamidfasern.

Die Farbstoffe (1) lassen sich auf verschiedene Weise auf das Fasermaterial applizieren und auf der Faser fixieren, insbesondere in Form von wäßrigen Farbstofflösungen und -druckpasten. Sie eignen sich sowohl für die allgemein bekannten Ausziehverfahren in weiten Temperaturbereichen, insbesondere bei 60 bis 80°C, als auch zum Färben nach dem Foulard-Färbeverfahren, wonach die Ware mit wäßrigen, gegebenenfalls salzhaltigen Farbstofflösungen imprägniert und der Farbstoff nach einer Alkalibehandlung oder in Gegenwart von Alkali, gegebenenfalls unter Wärmeeinwirkung, fixiert wird. Bevorzugt werden die Farbstoffe (1) in die Ausziehverfahren eingesetzt. Hierbei erhält man auch dann hochwertige Färbungen, wenn das Färbebad nur eine geringe Menge an einem oder mehreren Elektrolytsalzen, wie Natriumchlorid, Kaliumchlorid und Natriumsulfat, beispielsweise 20 bis 40 g/l Färbeflotte im Gegensatz zu den in der Technik allgemein verwendeten Mengen von 50 bis 80 g/l, enthält. Nach dem Fixieren werden die Färbungen oder Drucke mit kaltem und heißem Wasser, gegebenenfalls unter Zusatz eines dispergierend wirkenden und die Diffusion der nicht fixierten Anteile fördernden Mittels, gründlich gespült. Diese Färbe-und Druckverfahren sind zahlreich in der allgemeinen Fachliteratur wie auch in der Patentliteratur, wie beispielsweise in den anfangs genannten Druckschriften, beschrieben.

Gegenstand der vorliegenden Erfindung ist deshalb auch die Verwendung der Farbstoffe (1) zum Färben (einschließlich des Bedruckens) dieser Materialien bzw. Verfahren zum Färben (und Bedrucken) solcher Materialien in an und für sich üblicher Verfahrensweise, bei welchen ein Farbstoff (1) als Farbmittel eingesetzt wird, indem man den Farbstoff (1) im wäßrigen Medium auf das Material appliziert und ihn mittels Wärme oder mittels einer alkalisch wirkenden Verbindung oder mittels beidem auf dem Material fixiert.

Die Farbstoffe (1) zeichnen sich durch hohe Reaktivität, gutes Fixiervermögen und ein sehr gutes Aufbauvermögen aus. Die Fixiergrade sind hoch und nicht fixierte Anteile können leicht ausgewaschen werden, wobei die Differenz zwischen Ausziehgrad und Fixiergrad bemerkenswert klein, d.h. der Seifverlust sehr gering ist. Die Farbstoffe (1) eignen sich auch zum Druck, vor allem auf Baumwolle, ebenso aber auch zum Bedrucken von stickstoffhaltigen Fasern, zum Beispiel von Wolle oder Seide oder von Mischgeweben, die Wolle oder Seide enthalten.

Die mit den Farbstoffen (1) hergestellten Färbungen und Drucke besitzen, insbesondere auf Cellulosefasermaterialien, eine hohe Farbstärke und eine hohe Faser-Farbstoff-Bindungsstabilität sowohl in saurem als auch in alkalischem Bereich, weiterhin eine gute Lichtechtheit und sehr gute Naßechtheitseigenschaften, wie Wasch-, Wasser-, Seewasser-, Ueberfärbe- und Schweißechtheiten, sowie eine gute Plissierechtheit, Bügelechtheit und Reibechtheit.

Die nachfolgenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die in den Beispielen formelmäßig beschriebenen Verbindungen sind in Form der freien Säure angegeben; im allgemeinen werden sie in Form ihrer Alkalimetallsalze, wie Lithium-, Natrium- oder Kaliumsalze, hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, in die Synthese eingesetzt werden.

Die für die erfindungsgemäßen Farbstoffe angegebenen Absorptionsmaxima (λₘₐₓ) im sichtbaren Bereich wurden anhand ihrer Alkalimetallsalze in wäßriger Lösung ermittelt. In den Tabellenbeispielen sind die λₘₐₓ-Werte bei der Farbtonangabe in Klammern gesetzt; die Wellenlängenangabe bezieht sich auf nm.

### Beispiel 1

Eine Lösung des Lithiumsalzes von 58,9 Teilen 2,9-Diamino-9,13-dichlor-1,8-disulfo-triphendioxazin in 1500 Teilen Wasser wird bei 15 bis 20°C unter Einhaltung eines pH-Wertes zwischen 6 und 7 mittels einer Lithiumhydroxidlösung mit einer Suspension von 30 Teilen Cyanurchlorid in 200 Teilen Eiswasser versetzt. Man rührt den Ansatz noch kurze Zeit nach und gibt sodann 52 Teile N-Phenyl-N-γ-(β'-sulfatoethylsulfonyl)-propylamin als Lösung in 100 Teilen Wasser hinzu. Man erwärmt den Ansatz auf 50 bis 60°C, rührt noch einige Zeit unter Einhaltung eines pH-Wertes zwischen 4 und 5 nach und isoliert nach Beendigung der Umsetzung die erfindungsgemäße Triphendioxazinverbindung in üblicher Weise, beispielsweise durch Aussalzen mit Natriumchlorid als Alkalimetallsalz (Natriumsalz). Sie besitzt, in Form der freien Säure geschrieben, die Formel 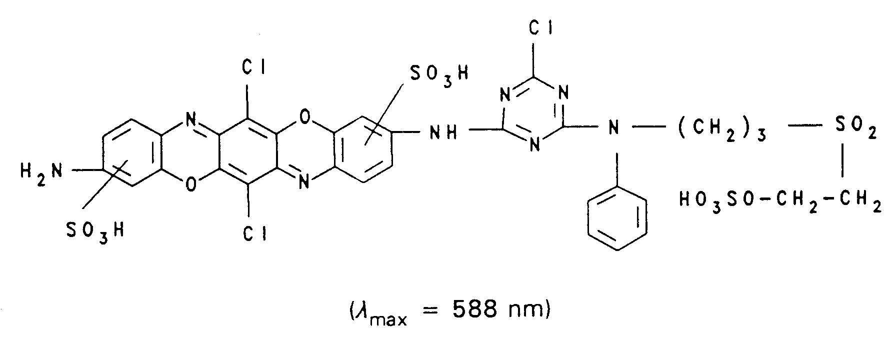 und zeigt sehr gute Farbstoffeigenschaften. Nach den in der Technik für faserreaktive Farbstoffe üblichen Applikations- und Fixierverfahren liefert sie auf den in der Beschreibung genannten Fasermaterialien, insbesondere Cellulosefasermaterialien, wie Baumwolle, sehr farbstarke, brillante, rotstichig blaue Färbungen und Drucke mit hoher Fixierquote.

### Beispiel 2

Die Synthese der in Beispiel 1 hergestellten Triphendioxazinverbindung kann auch so durchgeführt werden, daß man zunächst gemäß Beispiel 1 die Ausgangs-Diaminotriphendioxazin-Verbindung mit Cyanurchlorid umsetzt und die erhaltene Dichlortriazinylamino-triphendioxazin-Verbindung durch Aussalzen mit Natriumchlorid isoliert. Der feuchte Filterkuchen wird sodann in 500 Teilen Wasser gelöst; in diese Lösung wird eine Lösung von 33 Teilen N-Phenyl-N-γ-(β'-sulfatoethylsulfonyl)-propylamin in 100 Teilen Wasser eingerührt. Der Ansatz wird auf 50 bis 60°C erwärmt und noch einige Zeit unter Einhaltung eines pH-Wertes zwischen 4 und 5 nachgerührt. Die erfindungsgemäße Triphendioxazinverbindung wird in üblicher Weise isoliert. Sie besitzt dieselben guten Eigenschaften wie die gemäß den Angaben des Beispieles 1 hergestellte Triphendioxazinverbindung.

### Beispiel 3

Die erfindungsgemäße Triphendioxazin-Verbindung des Beispieles 1 läßt sich in deren Vinylsulfonyl-Derivat überführen, indem man beispielsweise 100 Teile der erfindungsgemäßen Triphendioxazin-Verbindung des Beispieles 1, in 1000 Teilen Wasser gelöst und bei 25°C mit soviel Natronlauge versetzt, bis der Ansatz einen pH-Wert von 12 erlangt hat. Man rührt den Ansatz unter Einhaltung dieses pH-Wertes und der angegebenen Temperatur noch 30 Minuten nach und isoliert sodann die erfindungsgemäße Verbindung der Formel (in Form der freien Säure geschrieben) 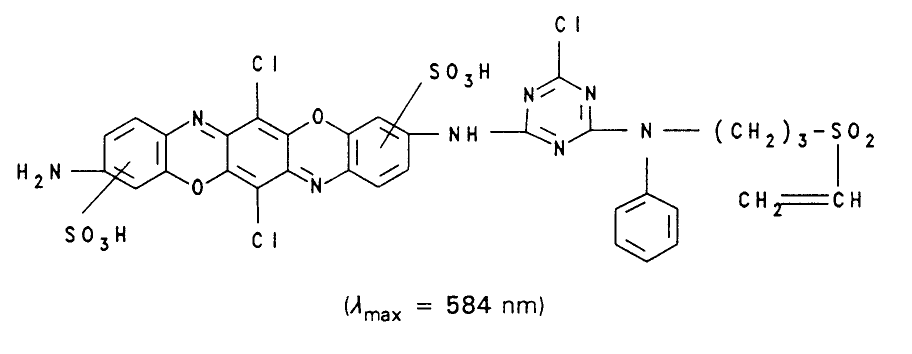 durch Aussalzen mit Natriumchlorid. Sie besitzt ebenfalls sehr gute faserreaktive Farbstoffeigenschaften und liefert, wie die erfindungsgemäße Verbindung des Beispieles 1, beispielsweise auf Cellulosefasern farbstarke, brillante, rotstichig blaue Färbungen und Drucke in hohen Fixierquoten.

### Beispiele 4 bis 8

In den nachfolgenden Tabellenbeispielen sind weitere erfindungsgemäße Triphendioxazin-Verbindungen entsprechend der allgemeinen Formel (A) 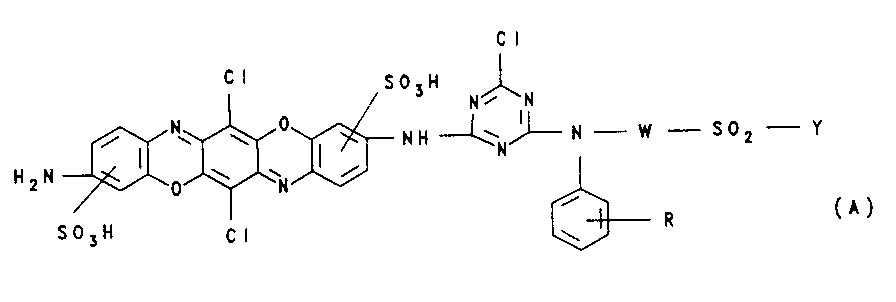 mit Hilfe der daraus ersichtlichen Komponenten beschrieben. Sie lassen sich in erfindungsgemäßer Weise, beispielsweise analog den obigen Beispielen, durch Umsetzung der aus den Formelresten von Formel (A) ersichtlichen Ausgangsverbindungen herstellen. Sie besitzen sehr gute faserreaktive Farbstoffeigenschaften und liefern auf den in der Beschreibung genannten Fasermaterialien, wie insbesondere Baumwolle, nach den für faserreaktive Farbstoffe üblichen Applikations- und Fixierverfahren farbstarke Färbungen und Drucke mit dem in den jeweiligen Tabellenbeispiele für Baumwolle angegebenen Farbton.

| Triphendioxazinverbindung (A) | | | | |
|---|---|---|---|---|
| Bsp. | Rest R | Rest W | Rest Y | Farbton |
| 4 | Wasserstoff | 2-Methyl-1,2-ethylen | β-Sulfatoethyl | rotstichig blau (585) |
| 5 | 3-Sulfo | 1,3-Propylen | β-Sulfatoethyl | rotstichig blau (582) |
| 6 | 4-Sulfo | 1,3-Propylen | β-Sulfatoethyl | rotstichig blau (586) |
| 7 | 3-Methyl | 1,3-Propylen | β-Sulfatoethyl | rotstichig blau (583) |
| 8 | 4-Methoxy | 1,3-Propylen | β-Sulfatoethyl | rotstichig blau (584) |

### Beispiel A

100 Teile eines Baumwollgewebes werden gemäß einem Auszieh-Färbeverfahren in einer üblichen Färbeapparatur mit guter Flottendurchdringung wie folgt gefärbt:
Zu einer in der Färbeapparatur befindlichen 25°C warmen Lösung von 1 Teil des erfindungsgemäßen Triphendioxazinfarbstoffes von Beispiel 1 und 30 Teilen Natriumchlorid in 1000 Teilen Wasser mit einem pH-Wert von 6 bis 7 gibt man 100 Teile eines Baumwollgewebes und bewegt es in dieser Färbeflotte zunächst während 10 Minuten. Man erhöht sodann die Temperatur der Färbeflotte innerhalb von 30 Minuten auf 80°C, setzt 2 Teile Natriumcarbonat hinzu und führt die Färbung während weiterer 20 Minuten bei 80°C fort. Danach gibt man nochmals 3 Teile Natriumcarbonat hinzu, färbt bei 80°C während 20 Minuten weiter, setzt nochmals 5 Teile Natriumcarbonat hinzu und färbt nochmals 20 Minuten bei 80°C und entnimmt sodann das gefärbt Gewebe aus der Färbeflotte. Es wird in üblicher Weise durch Spülen mit kaltem und warmem Wasser, durch kochende Behandlung in einem ein nichtionogenes Waschmittel enthaltenden Bad, durch erneutes Spülen mit warmem und kaltem Wasser und Trocknen fertiggestellt. Es wird eine sehr farbstarke, rotstichig blaue Färbung mit hohem Fixiergrad erhalten.

### Beispiel B

0,6 Teile des erfindungsgemäßen Triphendioxazinfarbstoffes von Beispiel 1 und 0,4 Teile des aus der europäischen Patentschrift Nr. 0 153 599 bekannten Triphendioxazinfarbstoffes der Formel 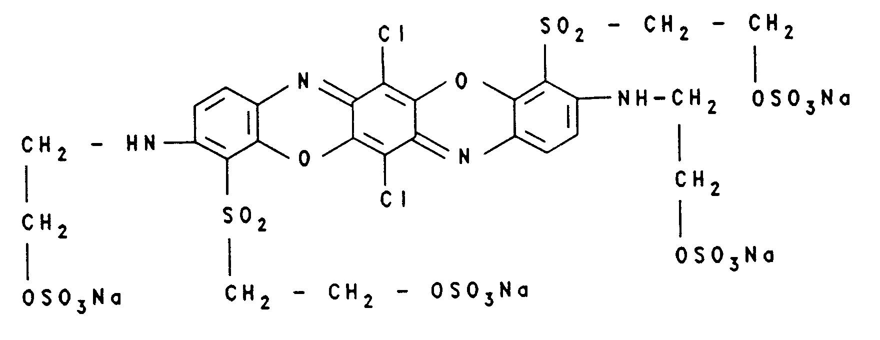 werden entweder als Mischung oder einzeln zusammen in 1000 Teilen Wasser gelöst. Zu dieser Farbstofflösung gibt man 30 Teile Natriumchlorid und färbt ein Baumwollgewebe gemäß den Angaben des Beispiels A.

Man erhält eine sehr farbstarke blaue Färbung, die eine kleine Nuance grünstichiger ist als die Färbung, die gemäß Beispiel A mit dem erfindungsgemäßen Triphendioxazinfarbstoff erhältlich ist.

## Patentansprüche

1. Triphendioxazin-Verbindung der allgemeinen Formel (1) 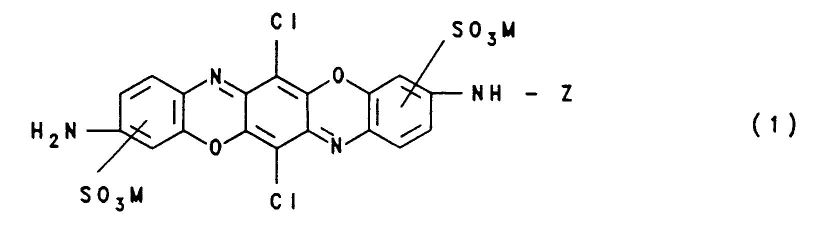 in welcher bedeuten:
M ist Wasserstoff oder ein Alkalimetall;
Z ist ein Rest der allgemeinen Formel (2) 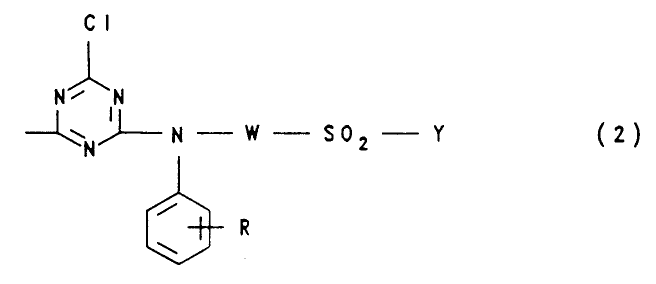
in welcher
R Wasserstoff, Sulfo, Alkyl von 1 bis 4 C-Atomen, Alkoxy von 1 bis 4 C-Atomen, Nitro oder Cyano ist,
W Alkylen von 3 bis 6 C-Atomen bedeutet, und
Y Vinyl ist oder Ethyl ist, das in β-Stellung durch einen bei Einwirkung von Alkali unter Bildung der Vinylgruppe eliminierbaren Substituenten substituiert ist.

2. Verbindung nach Anspruch 1, dadurch gekennzeichnet, daß R Wasserstoff oder Sulfo ist.

3. Verbindung nach Anspruch 1 oder 2, dadurch gekennzeichnet, daß W 1,3-Propylen oder 2-Methyl-1,2-ethylen ist.

4. Verbindung nach mindestens einem der Ansprüche 1 bis 3, dadurch gekennzeichnet, daß Y Vinyl, β-Chlorethyl oder β-Sulfatoethyl ist.

5. Verbindung nach mindestens einem der Ansprüche 1 bis 3, dadurch gekennzeichnet, daß Y β-Sulfatoethyl ist.

6. Verbindung nach Anspruch 1 der allgemeinen Formel 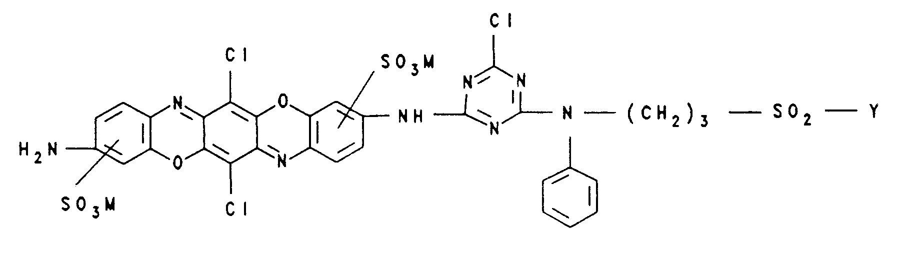 in welcher M und Y die in Anspruch 1 genannten Bedeutungen haben.

7. Verbindung nach Anspruch 6, dadurch gekennzeichnet, daß Y β-Sulfatoethyl ist.

8. Verfahren zur Herstellung einer Triphendioxazin-Verbindung der allgemeinen Formel (1) von Anspruch 1, dadurch gekennzeichnet, daß man eine Verbindung der allgemeinen Formel (3) 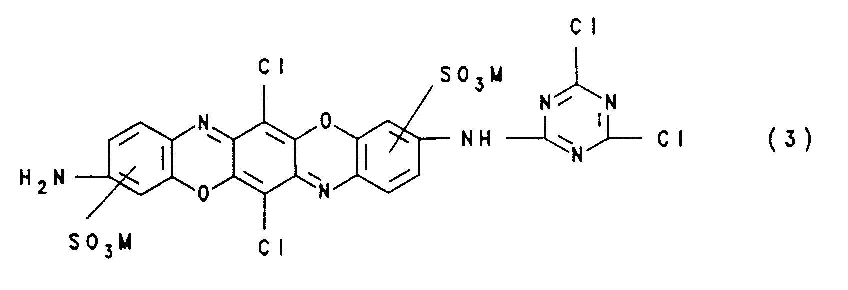 mit M der in Anspruch 1 genannten Bedeutung in wäßrigem oder wäßrigorganischem Medium bei einem pH-Wert zwischen 3 und 9 und einer Temperatur zwischen 25 und 100°C mit einer Aminoverbindung der allgemeinen Formel (4) 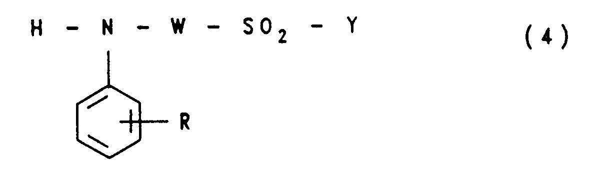 in welcher R, W und Y die in Anspruch 1 genannten Bedeutungen haben, umsetzt.

9. Mischung einer oder mehrerer Triphendioxazin-Verbindungen der allgemeinen Formel (1) von Anspruch 1 mit einer oder mehreren blaufärbenden Verbindungen anderer Konstitution aus der Disazo-, Kupferformazan, Triphendioxazin- und Anthrachinon-Reihe.

10. Verwendung einer Triphendioxazin-Verbindung der allgemeinen Formel (1) von Anspruch 1 oder einer Mischung von Anspruch 9 zum Färben von hydroxy- und/oder carbonamidgruppenhaltigem Material, insbesondere Fasermaterial.

11. Verfahren zum Färben von hydroxy- und/oder carbonamidgruppenhaltigem Material, vorzugsweise Fasermaterial, bei welchem man ein Farbstoff oder eine Farbstoffmischung in wäßrigem Medium mit dem Material in Kontakt bringt und die Fixierung des oder der Farbstoffe auf dem Material mittels Wärme oder mit Hilfe eines alkalisch wirkenden Mittels oder mit Hilfe beider Maßnahmen durchführt, dadurch gekennzeichnet, daß der Farbstoff oder die Mischung von Farbstoffen eine Triphendioxazin-Verbindung der allgemeinen Formel (1) von Anspruch 1 oder eine Mischung nach Anspruch 9 ist.

12. Verfahren nach Anspruch 11, dadurch gekennzeichnet, daß das Verfahren zum Färben ein Auszieh-Färbeverfahren ist und die Färbeflotte einen Elektrolytsalzgehalt von insgesamt 20 bis 40 g/l Färbeflotte enthält.

## Claims

1. A triphendioxazine compound of the formula (1) 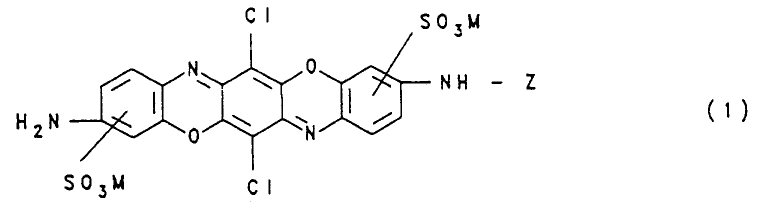 in which:
M is hydrogen or an alkali metal; and
Z is a radical of the formula (2) 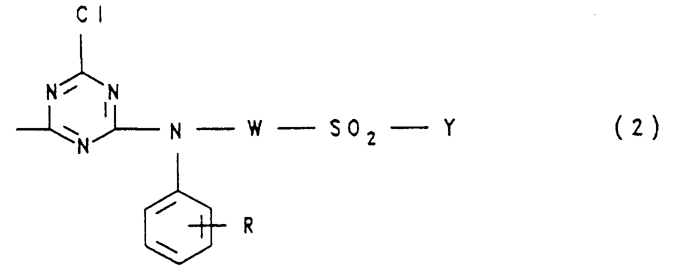
in which
R is hydrogen, sulfo, alkyl having 1 to 4 carbon atoms, alkoxy having 1 to 4 carbon atoms, nitro or cyano,
W is alkylene having 3 to 6 carbon atoms and
Y is vinyl, or is ethyl which is substituted in the β-position by a substituent which can be eliminated under the action of alkali to form the vinyl group.

2. A compound as claimed in claim 1, in which R is hydrogen or sulfo.

3. A compound as claimed in claim 1 or 2, in which W is 1,3-propylene or 2-methyl-1,2-ethylene.

4. A compound as claimed in at least one of claims 1 to 3, in which Y is vinyl, β-chloroethyl or β-sulfatoethyl.

5. A compound as claimed in at least one of claims 1 to 3, in which Y is β-sulfatoethyl.

6. A compound as claimed in claim 1 of the formula 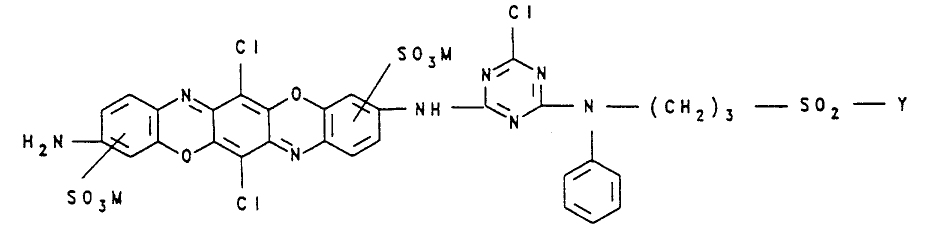 in which M and Y have the meanings given in claim 1.

7. A compound as claimed in claim 6, in which Y is β-sulfatoethyl.

8. A process for the preparation of a triphendioxazine compound of the formula (1) of claim 1, which comprises reacting a compound of the formula (3) 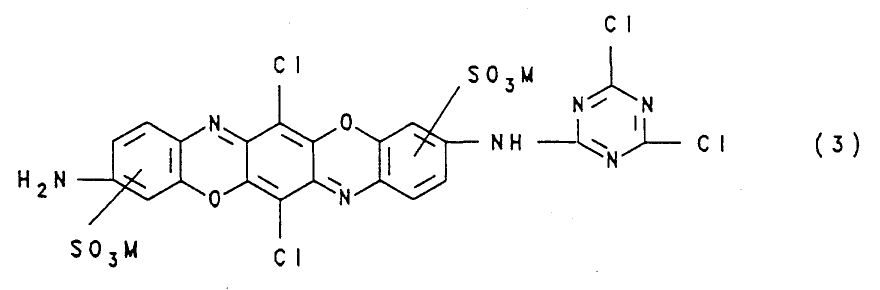 where M has the meaning given in claim 1, with an amino compound of the formula (4) 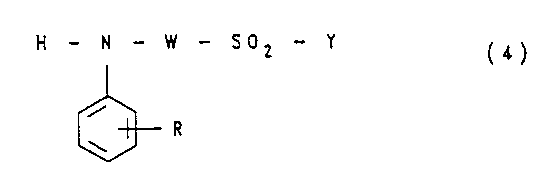 in which R, W and Y have the meanings given in claim 1, in an aqueous or aqueous-organic medium at a pH of between 3 and 9 and at a temperature of between 25 and 100°C.

9. A mixture of one or more triphendioxazine compounds of the formula (1) of claim 1 with one or more blue-dyeing compounds of different structure from the disazo, copper formazan, triphendioxazine and anthraquinone series.

10. The use of a triphendioxazine compound of the formula (1) of claim 1 or of a mixture of claim 9 for dyeing material, in particular fiber material, containing hydroxyl and/or carboxamide groups.

11. A process for dyeing material, preferably fiber material, containing hydroxyl and/or carboxamide groups, in which a dyestuff or a dyestuff mixture is brought into contact with the material in an aqueous medium and fixing of the dyestuff or dyestuffs on the material is carried out by means of heat or with the aid of an agent having an alkaline action or with the aid of both measures, wherein the dyestuff or the mixture of dyestuffs is a triphendioxazine compound of the formula (1) of claim 1 or a mixture as claimed in claim 9.

12. The process as claimed in claim 11, wherein the process for dyeing is an exhaustion dyeing process and the dye liquor has a total electrolyte salt content of 20 to 40 g/l of dye liquor.

## Revendications

1. Composé triphénodioxazine de formule générale (1) 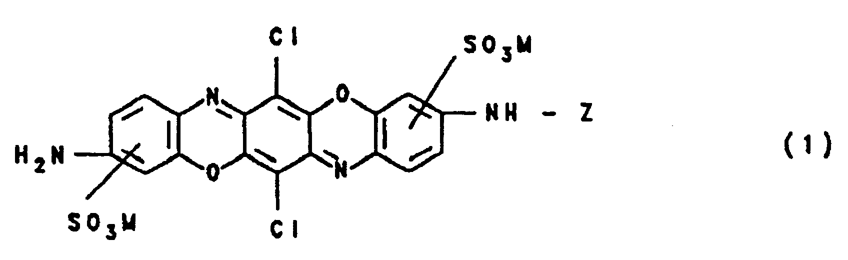 dans laquelle :
M est un hydrogène ou un métal alcalin ;
Z est un radical de formule générale (2) 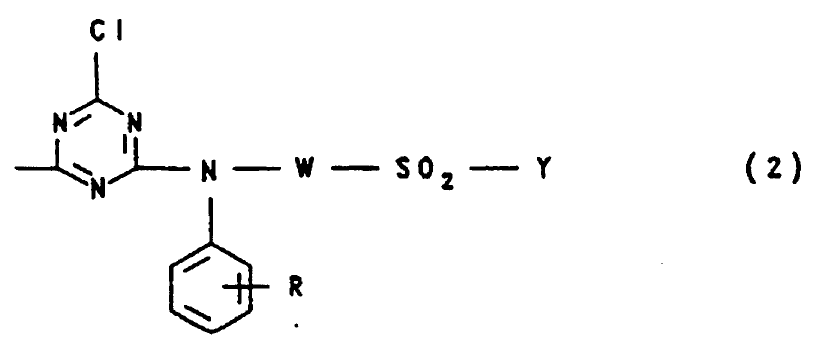
dans laquelle
R est un hydrogène, un sulfo, un alkyle ayant de 1 à 4 atomes de carbone, un alcoxy ayant de 1 à 4 atomes de carbone, un nitro ou un cyano,
W représente un alkylène ayant de 3 à 6 atomes de carbone, et
Y est un vinyle ou un éthyle qui est substitué en position β par un substituant pouvant être éliminé sous l'action d'un alcali en formant le groupe vinyle.

2. Composé selon la revendication 1, caractérisé en ce que R est un hydrogène ou un sulfo.

3. Composé selon la revendication 1 ou 2, caractérisé en ce que W est un 1,3-propylène ou un 2-méthyl-1,2-éthylène.

4. Composé selon au moins l'une des revendications 1 à 3, caractérisé en ce que Y est un vinyle, un β-chloroéthyle ou un β-sulfatoéthyle.

5. Composé selon au moins l'une des revendications 1 à 3, caractérisé en ce que Y est un β-sulfatoéthyle.

6. Composé selon la revendication 1, de formule générale 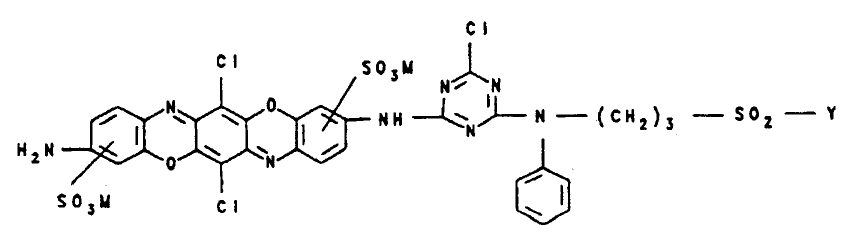 dans laquelle M et Y ont les significations mentionnées à la revendication 1.

7. Composé selon la revendication 6, caractérisé en ce que Y est un β-sulfatoéthyle.

8. Procédé de préparation d'un composé triphénodioxazine de formule générale (1) selon la revendication 1, caractérisé en ce que l'on fait réagir un composé de formule générale (3) 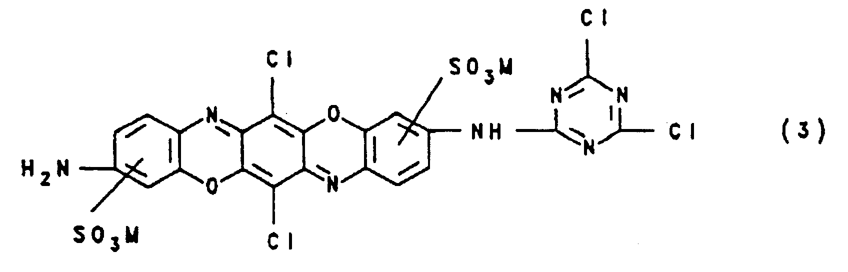 dans laquelle M a la signification mentionnée à la revendication 1, en milieu aqueux ou aqueux-organique à un pH compris entre 3 et 9 et une température comprise entre 25 et 100°C, avec un composé amino de formule générale (4) 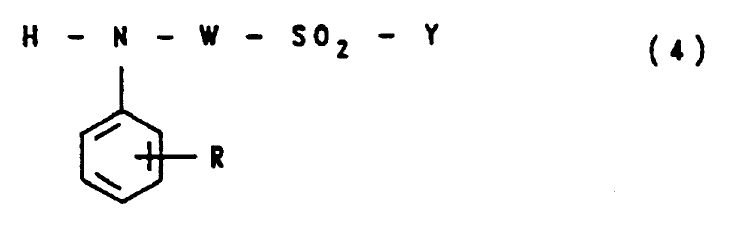 dans laquelle R, W et Y ont les significations mentionnées à la revendication 1.

9. Mélange d'un ou de plusieurs composés triphénodioxazine de formule générale (1) selon la revendication 1, avec un ou plusieurs composés de couleur bleue d'une autre constitution parmi la série des disazoïques, des formazanes au cuivre, des triphénodioxazines et des anthraquinones.

10. Utilisation d'un composé triphénodioxazine de formule générale (1) selon la revendication 1 ou d'un mélange selon la revendication 9, pour la teinture d'une matière renfermant des groupes hydroxy et/ou carbonamide, en particulier d'une matière fibreuse.

11. Procédé de teinture d'une matière renfermant des groupes hydroxy et/ou carbonamide, de préférence d'une matière fibreuse, dans lequel on amène un colorant ou un mélange de colorants en contact avec la matière en milieu aqueux et on réalise la fixation du ou des colorants sur la matière par la chaleur ou à l'aide d'un agent alcalin ou à l'aide des deux mesures, caractérisé en ce que le colorant ou le mélange de colorants est un composé triphénodioxazine de formule générale (1) selon la revendication 1 ou un mélange selon la revendication 9.

12. Procédé selon la revendication 11, caractérisé en ce que le procédé de teinture est un procédé de teinture à épuisement et la liqueur de teinture présente une teneur en sel électrolytique de 20 à 40 g/l de liqueur de teinture au total.
